# EUROPÄISCHE PATENTSCHRIFT

(11) **EP 2 295 617 B1**
(45) Veröffentlichungstag und Bekanntmachung des Hinweises auf die Patenterteilung: **29.02.2012**
(21) Anmeldenummer: 10015574.6
(22) Anmeldetag: 12.03.2008
(51) Int. Cl.: C23C 18/08, C23C 18/12, C23C 18/14, G02B 1/12, G02B 3/00

(54) **Verfahren zur Herstellung von flächigen Größen- oder Abstandsvariationen in Mustern von Nanostrukturen auf Oberflächen**
Method for producing flat size or distance variations in nanostructures on surfaces
Procédé de fabrication de variations à plat de grandeurs ou de distances dans des motifs de nanostructures sur des surfaces

(30) Priorität: 11.04.2007 DE 102007017032
(43) Veröffentlichungstag der Anmeldung: 16.03.2011
(62) Teilanmeldung aus: 08716484.4
(73) Patentinhaber: Max-Planck-Gesellschaft zur Förderung der Wissenschaften e.V., 80539 München (DE)
(72) Erfinder: Spatz, Joachim P., 70569 Stuttgart (DE); Lohmüller, Theobald, 90473 Nürnberg (DE); Arnold, Marco, 69251 Gaiberg (DE)
(74) Vertreter: Katzameyer, Michael

(56) Entgegenhaltungen:
- EP-B- 1 027 157
- US-A- 5 891 366
- US-A1- 2002 145 132
- US-A1- 2005 059 760
- L. ZHOU ET AL: "Scanning thermal microscopy and atomic force microscopy studies of laer-induced deposited metal lines", APPLIED SURFACE SCIENCE, Bd. 120, 1997, Seiten 149-158, XP002523305,
- D. POONDI ET AL: "Synthesis of silver nanoparticles by a laser-liquid-solid interaction technique", JOURNAL OF MATERAILS SYNTHESIS AND PROCESSING, Bd. 6, Nr. 2, 1998, Seiten 89-104, XP002523306,

## Beschreibung

Die Darstellung von chemischen und topographischen Variationen auf der Nano- und Mikrometerskala sind unter anderem für Anwendungen auf den Gebieten der refraktiven und diffraktiven Optik, der Selektion molekular wichtiger Längenskalen bei der Zelladhäsion und der Einzelmolekülanbindung an Grenzflächen von großem technischen Interesse. Dies gilt insbesondere für graduelle Veränderungen der Topographie und chemischen Eigenschaften einer Oberfläche.

Die Erzeugung von molekular wohl definierten Gradienten bietet z.B. die Möglichkeit, richtungsgesteuerte Prozesse wie die Zellbewegung, die externe chemische Gradienten erfordert, zu untersuchen. Besonders auf dem Gebiet der zellulären Haptotaxie sind chemische Gradienten mit molekularer Präzision wünschenswert, um biologische Reaktionen qualitativ und quantitativ detailliert zu untersuchen. Efimenko und Genzer ((Adv. Mater. 13, 2001), 1560-1563) haben gezeigt, dass eine Feinstellung der Pfropfdichte von Molekülen auf Oberflächen durch die Herstellung mechanisch assemblierter Monoschichten (MAMs) möglich ist. MAMS sind Strukturen, welche durch eine Kombination von Selbstassemblierung, mechanischer Streckung eines PDMS-Trägers und molekularer Diffusion hergestellt werden. Das Verfahren zur Herstellung dieser MAMs ist durch die Inkorporation eines mechanischen Schrittes relativ aufwendig und darüber hinaus nur für flexible Träger und nicht für starre Substrate, wie z.B. Glas, Metall, Siliciumverbindungen etc., geeignet. Einen weiteren Nachteil stellt die Tatsache dar, dass eine Molekül-Molekül-Abstandsregulierung durch mechanisches Strecken eines Substrats nicht möglich ist, da das Clustern von einzelnen Molekülen nicht wesentlich eingeschränkt werden kann.

Vor diesem Hintergrund besteht eine Aufgabe der vorliegenden Erfindung in der Bereitstellung eines einfachen und vielseitig anwendbaren Verfahrens mit dem flächige Größenvariationen in Mustern von Nanostrukturen auf Oberflächen so präzise eingestellt werden können, dass definierte Gradientenoberflächen erhalten werden.

Diese Aufgabe wurde erfindungsgemäß gelöst durch eine Weiterentwicklung der mizellaren Nanolithographie (siehe z.B. EP 1 027 157). Bei der mizellaren Nanolithographie wird eine mizellare Lösung eines Blockcopolymers auf ein Substrat abgeschieden, z.B. durch Tauchbeschichtung, und bildet unter geeigneten Bedingungen auf der Oberfläche eine geordnete Filmstruktur von chemisch unterschiedlichen Polymerdomänen, die unter anderem von Typ, Molekulargewicht und Konzentration des Blockcopolymers abhängt. Die Mizellen in der Lösung können mit anorganischen Salzen beladen werden, die nach der Abscheidung mit dem Polymerfilm zu anorganischen Nanopartikeln reduziert werden können. Es wurde nun erfindungsgemäß festgestellt, dass die Größe dieser Nanopartikel durch Bestrahlung des abgeschiedenen Polymerfilms mit einem lateral intensitätsmodulierten Lichtfeld so präzise flächig eingestellt werden kann, dass nanostrukturierte Oberflächen mit gewünschten Größengradienten herstellbar sind.

EP 1027157 B1 offenbart ein Verfahren, bei dem eine mizellare Lösung eines Polymers gebildet wird, die Mizellen mit einer anorganischen Metallverbindung beladen und dann als Film auf einer Substratoberfläche abgeschieden werden, wobei die ursprüngliche Metallverbindung vor dem Aufbringen der Mizellen auf die Substratoberfläche durch energiereiche Strahlung in das Metall oder ein Metalloxid überführt wird, und wobei in einem letzten Schritt das Polymer entfernt wird und eine regelmäßige Struktur von Metallclustern auf der Oberfläche zurück bleibt. Zhou et al. beschreiben in Applied Surface Science 120 (1997) 149-158) ein Verfahren zur laserinduzierten Abscheidung von Palladium aus Palladiumacetat auf einer Glasoberfläche. Eine gezielte Größenvariation der Metallcluster bzw. Partikel durch eine flächig lateral intensitätsmodulierte Bestrahlung wird in keiner der beiden Druckschriften beschrieben oder vorgeschlagen.

Gegenstand der vorliegenden Erfindung sind somit Verfahren zur flächigen Größenvariation in Mustern von Nanostrukturen auf einem Substrat gemäß dem unabhängigen Anspruch 1. Spezielle oder bevorzugte Ausführungsformen der Erfindung sind Gegenstand der abhängigen Ansprüche.

### Beschreibung der Erfindung

Wie bereits oben erwähnt, beruht die vorliegende Erfindung auf einer Weiterentwicklung der Technik der mizellaren Nanolithographie (siehe z.B. EP 1 027 157). Bei der mizellaren Nanolithographie wird eine mizellare Lösung eines Diblock- oder Multiblockcopolymers auf ein Substrat abgeschieden, z.B. durch Tauchbeschichtung, und bildet unter geeigneten Bedingungen auf der Oberfläche eine geordnete Filmstruktur von chemisch unterschiedlichen Polymerdomänen, die unter anderem von Typ, Molekulargewicht und Konzentration des Blockcopolmers abhängt. Beispielsweise sind die Abstände der einzelnen Polymerdomänen voneinander abhängig vom Molekulargewicht und der Konzentration des Blockcopolymers in der Lösung. Die Mizellen in der Lösung können mit anorganischen Salzen bzw. Säuren beladen werden, die nach der Abscheidung mit dem Polymerfilm zu anorganischen Nanopartikeln reduziert werden können.

Es wurde nun festgestellt, dass sowohl die laterale Separationslänge der genannten Polymerdomänen und damit auch der resultierenden Nanopartikel als auch die Größe dieser Nanopartikel durch verschiedene Maßnahmen so präzise flächig eingestellt werden können, dass nanostrukturierte Oberflächen mit gewünschten Abstands- und/oder Größengradienten herstellbar sind.

Als Zweiblock- oder Multiblockcopolymer in diesem Verfahren kann grundsätzlich jedes Mizellen-bildende Blockcopolymer verwendet werden, welches als Film auf ein Substrat abgeschieden werden kann und dabei eine geordnete Struktur verschiedener Polymerdomänen bildet. Geeignete Blockcopolymere sind beispielsweise alle in der oben zitierten EP 1 027 157 genannten Blockcopolymere. In einer spezielleren Ausführungsform ist das Zweiblock- oder Multiblockcopolymer aus der Gruppe aus Polystyrol(n)-b-Poly(2-vinylpyridin(m), Polystyrol(n)-b-Poly(4-vinylpyridin(m), Polystyrol(n)-b-Poly(ethylenoxid)(m), wobei n und m die Anzahl der Wiederholungseinheiten angeben und unabhängig voneinander ganze Zahlen im Bereich von 10-10000, insbesondere 100-1000, sind, ausgewählt. Vorzugsweise wird das Molekulargewicht (Mw) (gelöster Block) >> Mw (schlecht gelöster Block) gewählt.

Als anorganische Verbindungen, mit denen die Mizellen in der Lösung bzw. die Polymerdomänen in dem abgeschiedenen Kunststofffilm beladen werden können, sind grundsätzlich alle anorganischen Metallverbindungen (z.B. Metallsalze) geeignet, die durch Oxidation oder Reduktion in anorganische Nanopartikel überführt werden können. Geeignete Salze sind beispielweise alle in der oben zitierten EP 1 027 157 genannten Metallsalze. Vorzugsweise umfassen die erfindungsgemäß verwendeten Metallsalze mindestens ein Salz der Metalle Au, Pt, Pd, Ag, In, Fe, Zr, Al, Co, Ni, Ga, Sn, Zn, Ti, Si oder Ge. Besonders bevorzugt ist HAuCl₄.

Gegenstand der vorliegenden Erfindung ist ein Verfahren zur Erzeugung von Nanopartikeln mit einer lateral variablen Größe auf einer Substratoberfläche, bei dem ein Polymerfilm, der mindestens ein anorganische Metallverbindung (z.B. ein Metallsalz) enthält, auf einer Substratoberfläche abgeschieden wird und dann mit einem flächig lateral intensitätsmodulierten Lichtfeld bestahlt wird, wodurch das Metallsalz reduziert und in Nanopartikel überführt wird und die Größe der Nanopartikel an einer bestimmten Position auf der Substratoberfläche abhängig von der Intensität des Lichtfelds an dieser Position ist, wobei der Polymerfilm durch Abscheidung einer mizellaren Lösung eines organischen Zweiblock- oder Multiblockcopolymers, deren Mizellen mit mindestens einer organischen Metallverbindung beladen sind, auf der Substratoberfläche gebildet wird.

Auf diese Weise kann ein Gradient der Partikelgröße auf der Substratoberfläche erzeugt werden.

Vorzugsweise wird der Polymerfilm dabei auf der Substratoberfläche so abgeschieden, dass eine geordnete Struktur von metallsalzhaltigen Polymerdomänen mit einer bestimmten lateralen Separationslänge erzeugt wird, wobei diese Separationslänge nach der Überführung der Metallverbindungen in Nanopartikel die Separationslänge der Nanopartikel bestimmt. In einer spezielleren Ausführungsform dieses Verfahren wird der Polymerfilm so abgeschieden, dass auf der Substratoberfläche ein Separationslängen-Gradient von Polymerdomänen erzeugt wird.

Vorzugsweise wird das Substrat mit dem abgeschiedenen Polymerfilm nach der Bestrahlung mit Licht einer Plasmabehandlung, z.B. mit Sauerstoff- oder Wasserstoffplasma, unterzogen, die das Polymer ganz oder teilweise abbaut.

In einer spezielleren Ausführungsform dieses Verfahrens wird die Substratoberfläche mit den gebildeten Nanopartikeln anschließend in eine flüssige Phase eingebracht, die eine Lösung einer Metallverbindung enthält, wobei gelöstes Material auf den Nanopartikeln aufwächst und diese damit vergrößert, und das Substrat aus der flüssigen Phase nach einer vorbestimmten Zeitspanne herausgezogen wird, wodurch eine gewünschte Größe der Nanopartikel eingestellt wird.

Bei dem erfindungsgemäßen Verfahren, in dem eine Bestrahlung mit intensitätsmoduliertem Licht vorgenommen wird, kann die Intensität des Lichtfeldes flächig graduell oder stufenweise durch Interferenz und Beugung an statischen oder dynamisch veränderbaren optischen Elementen eingestellt werden. Eine typische Vorrichtung hierfür umfasst z.B. folgende Komponenten: Lichtquelle, Spiegel, Interferenzgitter, diffraktive optische Elemente. Letztere können im Falle von Flüssigkristallen oder Mikrospiegeln musteraktiv geschaltet werden und so hinsichtlich ihrer Interferenzeigenschaften aktiv (dynamisch) beeinflusst werden.

### Kurzbeschreibung der Figuren

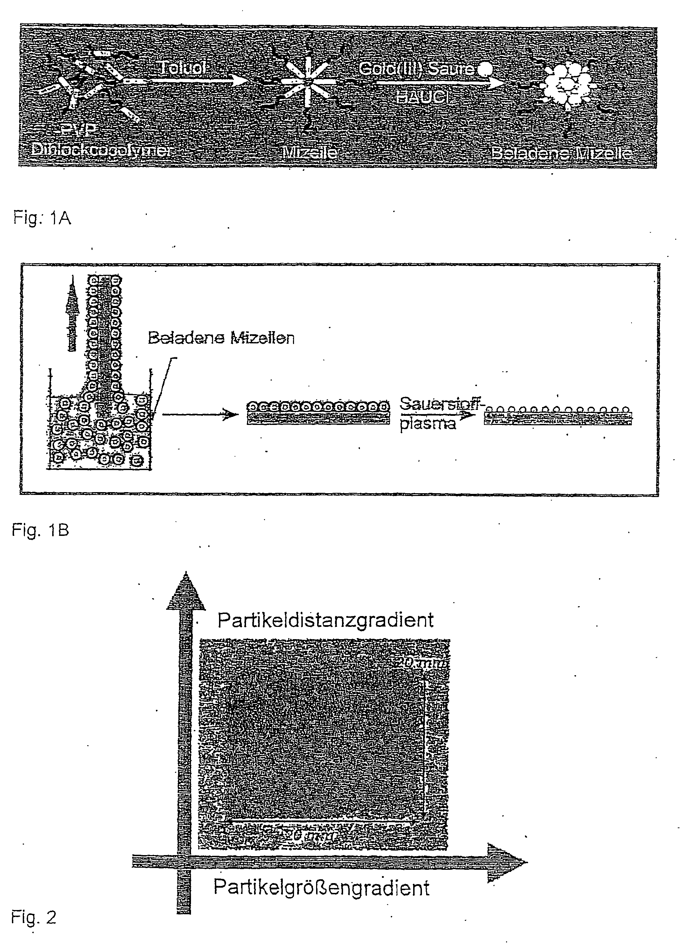

**Fig. 1A** zeigt schematisch die Bildung einer Mizelle aus einem PS-PVP-Diblockcopolymer und die Beladung mit einer Gold(III)-Verbindung.
**Fig. 1B** zeigt schematisch die Aufbringung beladener Mizellen auf ein Substrat und die spätere Entfernung des Polymerfilms durch eine Plasmabehandlung.
**Fig. 2** zeigt eine Photographie eines zweifachen Gradienten aus Gold-Nanopartikeln.

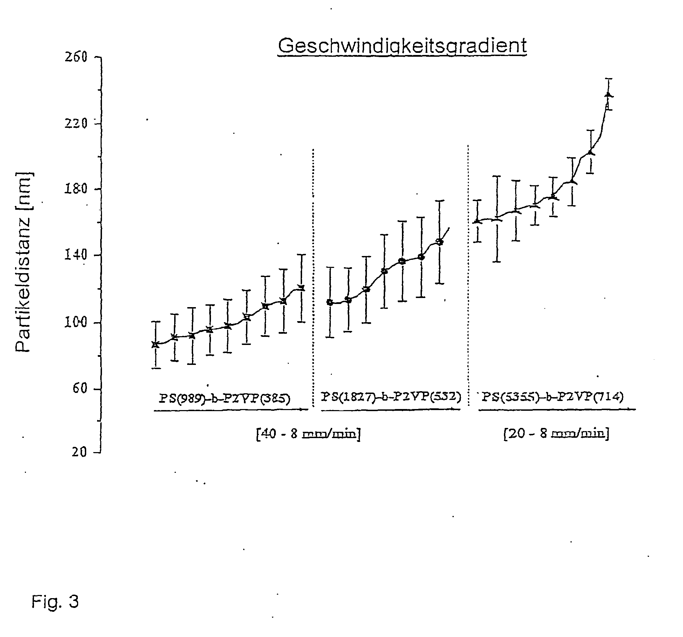

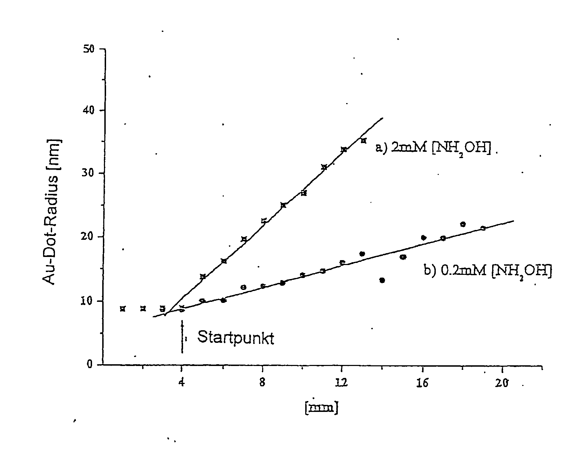

**Fig. 3** zeigt die Variation des Partikelabstands als Funktion der Ziehgeschwindigkeit eines Substrats aus einer mizellaren Lösung.
**Fig. 4 A-D** zeigen die mittleren Au-Goldpartikel-Separationslängen, die bei verschiedenen Ziehgeschwindigkeiten für verschiedene Copolymerlösungen A-D erhalten wurden.
**Fig. 5** zeigt die Korrelation zwischen dem Ordnungsparameter, den Interpartikeldifferenzen und den Ziehgeschwindigkeiten für diese Lösungen A-D.
**Fig. 6** zeigt repräsentative rasterelektronenmikroskopische Aufnahmen der Nanomuster, die von verschiedenen Separationslängengradienten bei einer Position am Anfang des Gradienten, als das Substrat mit hoher Ziehgeschwindigkeit aus der entsprechenden Blockcopolymerlösung herausgezogen wurde (A1, B1, C1), und einer Position am Ende des Gradienten mit einer niedrigen Ziehgeschwindigkeit (A2, B2, C2) erhalten wurden.
**Fig. 7** zeigt die entlang der Gradienten gemessenen Interpartikeldistanzen (Fig 7A-C) und die Gestaltung der Gradienten (Fig. 8D).
**Fig. 8** gibt die Interpartikeldistanzen von zwei konvers orientierten Gradienten auf einem Substrat als Plot gegen die Eintauchtiefe an und zeigt rasterelektronenmikroskopische Aufnahmen von verschieden Positionen auf jedem Substrat.

Die folgenden Beispiele dienen zur näheren Erläuterung wie im Rahmen des Verfahrens der vorliegenden Erfindung neben der Größenvariation der Nanopartikel eine zusätzliche Abstandsvariation der Nanopartikel erzeugt werden kann.

### BEISPIEL 1

### Abstandsvariation von Gold-Nanopartikeln durch die Verwendung verschiedener konstanter Geschwindigkeiten, um das Substrat aus einer mizellaren Lösung zu ziehen

Es wurden verschiedene Lösungen A, B, C und D hergestellt, deren Charakteristiken in der folgenden Tabelle 1 zusammenfasst sind.

**Tabelle 1**

| Mizellare Lösung | Polymer | L = HauCl₄/P2VP | Polymer-Konz. ( mg/ml) |
|---|---|---|---|
| A | PS(1780)-b-P2VP(520) | 0,2 | 1 |
| B | PS(1780)-b-P2VP(520) | 0,2 | 2 |
| C | PS(990)-b-P2VP(385) | 0,3 | 3 |
| D | PS(990) -b-P2VP(385) | 0,3 | 5 |

Diese Lösungen wurden hergestellt durch Lösen des jeweiligen Blockcopolymers in der vorgesehenen Konzentration in trockenem Toluol und anschließendes Zugeben von HAuCl₄ x H₂O im angegebenen Verhältnis L, wobei die Mischung solange gerührt wurde, bis die Tetrachlorgoldsäure vollständig solubilisiert war.

Für jede Lösung wurden 10 Proben (Glas-Objektträger) sorgfältig gereinigt, z.B. mit "Piranha"-Lösung (H₂O₂/H₂SO₄ = 1:1), mit reinem Wasser getrocknet, in einem Stickstoffstrom getrocknet und in die jeweilige mizellare Lösung getaucht und mit verschiedenen Geschwindigkeiten herausgezogen. Nach einer Wasserstoff-plasmabehandlung (z.b. wie in EP 1 027 157 beschrieben) wurden die Gold-Nanopartikel-Distanzen mit dem Rasterelektronenmikroskop bestimmt. Hierfür wurden 5 verschiedene Bereiche auf jedem Glasträger (20 x 20 mm) bei einer Vergrößerung von 5000 analysiert.

Zur Berechnung der jeweiligen mittleren Interpartikeldistanzen und der Standardabweichungen wurde das Computer-Analyseprogramm Origin 7.0 eingesetzt. Die 5 erhaltenen Nanopartikel-Separationswerte mit ihren jeweiligen Standardabweichungen wurden gemittelt, um die mittleren Gesamt-Interpartikeldistanzen für einen einzelnen Objektträger, der mit einer bestimmten Ziehgeschwindigkeit aus der Lösung des Diblockcopolymers herausgezogen wurde, für jede der 10 verschiedenen Ziehgeschwindigkeiten zu ermitteln.

**Tabelle 2**

| Mizellare Lösung | Minimale Interpartikeldistanz (nm) ± Standardabweichung | Minimale Interpartikeldistanz (nm) ± Standardabweichung | Δ (nm) |
|---|---|---|---|
| A | 128 ± 18 | 175 ± 28 | 47 |
| B | 93 ± 13 | 125 ± 18 | 32 |
| C | 58 ± 9 | 93 ± 14 | 35 |
| D | 59 ± 9 | 94, ± 14 | 35 |

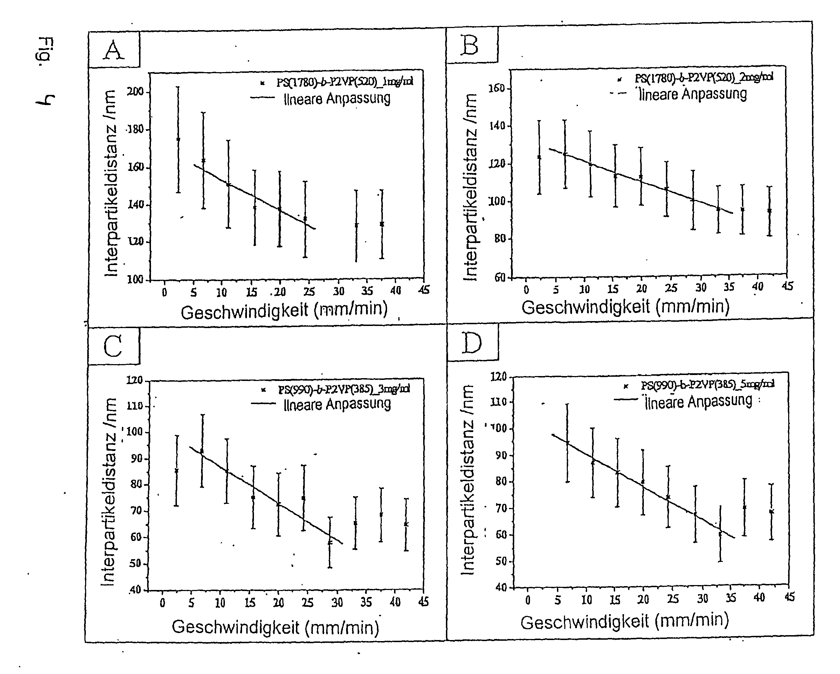

Fig. 4A-D zeigt alle getesteten Lösungen und die jeweiligen korrespondierenden Interpartikeldistanzen bei jeder Ziehgeschwindigkeit. Diese Studie demonstrierte, dass in allen getesteten Lösungen eine nahezu lineare Abnahme der Distanzen mit höheren Ziehgeschwindigkeiten erhalten werden konnte. Der maximale Unterschied in der lateralen Beabstandung der Gold-Nanopartikel für die getesteten Lösungen war etwa 40 nm. Es ist bemerkenswert, dass bei einer bestimmten Geschwindigkeit die Beabstandung der Gold-Nanopartikel eine Sättigung zu erfahren scheint. Anderseits wurde eine Abnahme der Musterordnung bei einer sehr geringen Geschwindigkeit (2,3 mm/min) beobachtet, die zu stark differierenden Interpartikeldistanzen an verschiedenen Messpositionen auf dem Substrat führte.

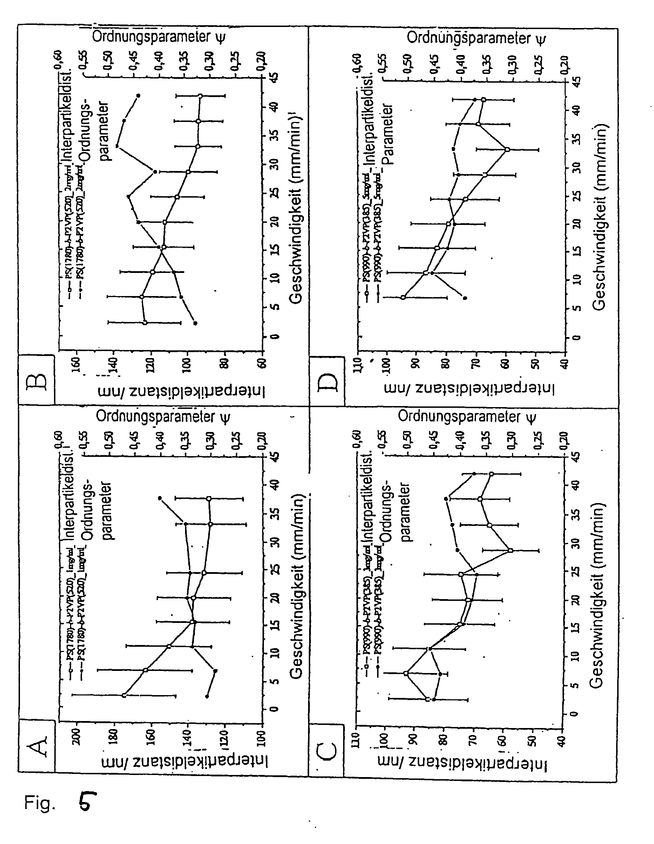

Fig. 5 zeigt die Korrelation zwischen dem Ordnungsparameter, den Interpartikeldifferenzen und den Ziehgeschwindigkeiten. Dabei zeigten die Lösungen A und B eine Erhöhung des Ordnungsparameters mit zunehmender Geschwindigkeit, während der Ordnungsparameter konstant blieb oder leicht abnahm, wenn die Ziehgeschwindigkeit in den Lösungen C und D erhöht wurde. Offenbar führt eine zu niedrige Mizellendichte auf der Oberfläche (z.B. aufgrund einer zu niedrigen Polymerkonzentration oder einer zu niedrigen Ziehgeschwindigkeit) zu einem geringeren Ordnungsgrad auf der Oberfläche und es kann gefolgert werden, dass es für jedes Diblockcopolymer eine optimale Mizellendichte in einer monomizellaren Schicht bezüglich der Musterqualität gibt.

### BEISPIEL 2

### Separationslängengradienten von Gold-Nanopartikeln durch Variation der Ziehgeschwindigkeit des Substrats aus einer mizellaren Lösung

Bei diesem Experiment wurden drei Proben mit Gradienten von 1 mm, 2 mm und 3 mm Länge hergestellt. Die Variation der Ziehgeschwindigkeit aus der mizellaren Lösung, hergestellt analog zu Beispiel 1, variierte von 40 mm/min ("Ausgangsgeschwindigkeit") bis 8 mm/min ("Endgeschwindigkeit").

Die Substrate wurden mit einem Elektromotor bewegt, dessen Ziehgeschwindigkeit über die programmierbare Spannung seiner Energieversorgung eingestellt werden konnte.

**Tabelle 3**

| | Polymer | L = HauCl₄/P2VP | Polymer-Konz. (mg/ml) | Gradientenlänge (mm) | Steigung (nm/mm) |
|---|---|---|---|---|---|
| Gradient 1 | PS(990) -*b*-P2VP(385) | 0,3 | 3 | 1 | 21 ± 9 |
| Gradient 2 | PS(990)-*b*-P2VP(385) | 0,3 | 3 | 2 | 10 ± 6 |
| Gradient 3 | PS(990)-*b*-P2VP(385) | 0,3 | 5 | 3 | 7 ± 4 |

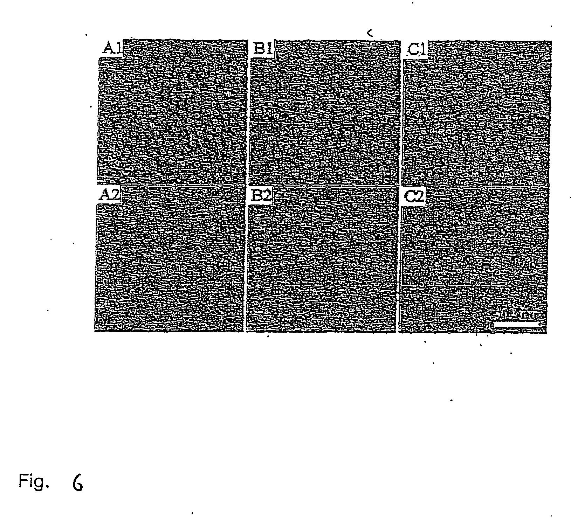

Fig. 6 zeigt rasterelektronenmikroskopische Aufnahmen der Nanomuster, die von jedem Gradientensubstrat bei einer Position am Anfang des Gradienten, als das Substrat mit 40 mm/min Ziehgeschwindigkeit aus der entsprechenden Blockcopolymerlösung herausgezogen wurde (A1, B1, C1), und einer Position am Ende des Gradienten mit einer Ziehgeschwindigkeit von 8 mm/min (A2, B2, C2) erhalten wurden (A Gradient 1; B Gradient 2; C Gradient 3). Die Rasterelektronenmikroskop-Aufnahmen demonstrieren, dass keine Beeinträchtigung der Gold-Nanomuster festzustellen ist, wenn ein einziges Substrat mit den angegebenen verschiedenen Geschwindigkeiten herausgezogen wird.

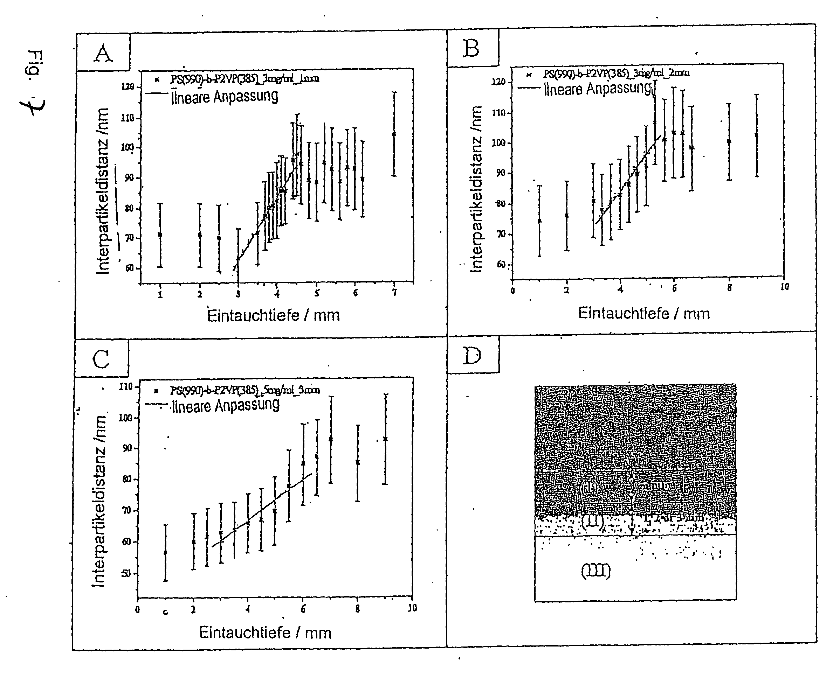

Fig. 7 zeigt die entlang der Gradienten gemessenen Interpartikeldistanzen (Fig 7A-C) und die Gestaltung der Gradienten (Fig. 7D). Aus Fig 7A-C ist ersichtlich, dass eine nahezu lineare Zunahme der Interpartikeldistanten auftrat, welche der linearen Abnahme der Ziehgeschwindigkeit zuzuschreiben ist. Dies bedeutet, dass für kürzere Gradientenlängen steilere Steigungen erhalten wurden. Die Steigung eines Gradienten ist durch das Verhältnis der Gradientenlänge zur Zunahme der Interpartikeldistanzen angegeben. Alle Gradienten begannen und endeten an Positionen, welche durch ein Computerprogramm vorgegeben wurden.

### BEISPIEL 3

### Herstellung von mehreren Separationslängengradienten auf einem Substrat

In diesem Experiment wurden zwei verschiedene Gradienten auf einem Substrat erzeugt. Dazu wurde die Ziehgeschwindigkeit für den ersten Gradienten von 8 auf 40 mm/min erhöht und für den zweiten Gradienten von 40 auf 8 mm/min verringert.

**Tabelle 4**

| Eigenschaften zweier konvers orientierter Gradienten | | | | | | |
|---|---|---|---|---|---|---|
| | Polymer | L = HauCl₄/P2VP | Polymer-Konz. (mg/ml) | Gradientenlänge (mm) | Steigungen (nm/mm) | |
| | | | | | 1 | 2 |
| Gradient 4 | PS(1780)-*b*-P2VP(520) | 0,2 | 2 | 3 | - 11 ± 5 | + 11 ± 5 |
| Gradient 5 | PS(990)-*b*-P2VP(385) | 0,3 | 3 | 1 | -31 ±8 | 23 ± 9 |

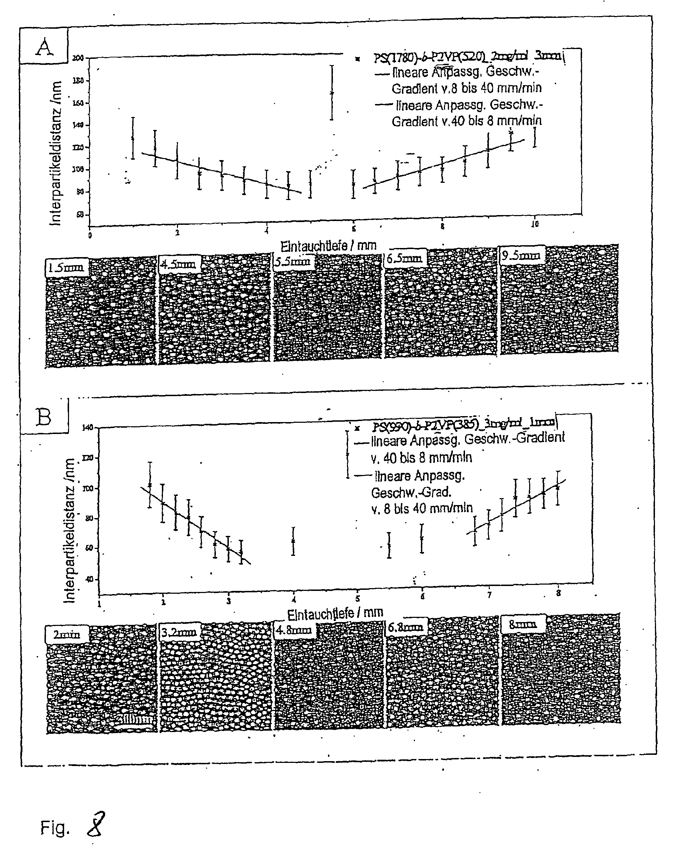

Fig. 8 gibt die Interpartikeldistanzen dieser konvers orientierten Gradienten als Plot gegen die Eintauchtiefe (entspricht der Gradientenlänge) an und zeigt rasterelektronenmikroskopische Aufnahmen von verschiedenen Positionen auf jedem Substrat.

Die Bestimmung der Interpartikeldstanzen ergab Resultate, die mit denjenigen von Einzelgradienten vergleichbar waren. Diese Ergebnisse belegen, dass durch Änderung der Ziehgeschwindigkeit eine Gradientenbildung in beiden Richtungen möglich war. Die ungewöhnlich hohen Interpartikeldistanzen, die in der Mitte des Substrats beobachtet wurden, können durch den plötzlichen Stopp auf 0/min von einer Geschwindigkeit von 40 mm/min erklärt werden und können als Bereiche betrachtet werden, die mit sehr geringer Geschwindigkeit herausgezogen wurden.

## Patentansprüche

1. Verfahren zur Erzeugung von Nanopartikeln mit einer lateral variablen Größe auf einer Substratoberfläche, bei dem ein Polymerfilm, der mindestens eine anorganische Metallverbindung enthält, auf einer Substratoberfläche abgeschieden wird und dann mit einem flächig lateral intensitätsmodulierten Lichtfeld bestrahlt wird, wodurch die Metallverbindung reduziert und in Nanopartikel überführt wird und die Größe der Nanopartikel an einer bestimmten Position auf der Substratoberfläche abhängig von der Intensität des Lichtfelds an dieser Position ist, wobei der Polymerfilm durch Abscheidung einer mizellaren Lösung eines organischen Zweiblock- oder Multiblockcopolymers, deren Mizellen mit mindestens einer organischen Metallverbindung beladen sind, auf der Substratoberfläche gebildet wird.

2. Verfahren nach Anspruch 1, **dadurch gekennzeichnet, dass** ein Gradient der Partikelgröße auf der Substratoberfläche erzeugt wird.

3. Verfahren nach Anspruch 1 oder 2, **dadurch gekennzeichnet, dass** der Polymerfilm auf der Substratoberfläche so abgeschieden wird, dass eine geordnete Struktur von metallverbindungshaltigen Polymerdomänen mit einer bestimmten lateralen Separationslänge erzeugt wird, wobei diese Separationslänge nach der Überführung der Metallverbindungen in Nanopartikel die Separationslänge der Nanopartikel bestimmt.

4. Verfahren nach Anspruch 3, **dadurch gekennzeichnet, dass** auf der Substratoberfläche ein Separationslängen-Gradient von Polymerdomänen erzeugt wird.

5. Verfahren nach einem der Ansprüche 1-4, **dadurch gekennzeichnet, dass** das Substrat mit dem abgeschiedenen Polymerfilm nach der Bestrahlung mit Licht einer Plasmabehandlung unterzogen wird, die das Polymer ganz oder teilweise abbaut.

6. Verfahren nach einem der Ansprüche 1-5, **dadurch gekennzeichnet, dass** die Substratoberfläche mit den gebildeten Nanopartikeln anschließend in eine flüssige Phase eingebracht wird, die eine Lösung einer anorganischen Metallverbindung enthält, wobei gelöstes Material auf den Nanopartikeln aufwächst und diese damit vergrößert, und das Substrat aus der flüssigen Phase nach einer vorbestimmten Zeitspanne herausgezogen wird, wodurch eine gewünschte Größe der Nanopartikel eingestellt wird.

7. Verfahren nach einem der Ansprüche 1-6 **dadurch gekennzeichnet, dass** die Intensität des Lichtfeldes flächig graduell oder stufenweise durch Interferenz und Beugung an statischen oder dynamisch veränderbaren optischen Elementen eingestellt wird.

## Claims

1. A method for creating nanoparticles with a laterally variable size on a substrate surface, in which a polymer film containing at least one inorganic metal compound is deposited on a substrate surface and is then irradiated with a light field laterally intensity-modulated in an extended area, as a result of which the metal compound is reduced and converted into nanoparticles and the size of the nanoparticles at a certain position on the substrate surface is dependent on the intensity of the light field at this position, wherein the polymer film is formed on the substrate surface by deposition of a micellar solution of an organic diblock or multiblock copolymer, the micelles of which are loaded with at least one organic metal compound.

2. Method according to Claim 1, **characterised in that** a gradient of the particle size is created on the substrate surface.

3. Method according to Claim 1 or 2, **characterised in that** the polymer film is deposited on the substrate surface in such a manner that an ordered structure of metal-compound-containing polymer domains with a certain lateral separation length is created, wherein this separation length determines the separation length of the nanoparticles following the conversion of the metal compounds into nanoparticles.

4. Method according to Claim 3, **characterised in that** a separation-length gradient of polymer domains is created on the substrate surface.

5. Method according to one of Claims 1-4, **characterised in that**, following the irradiation with light, the substrate with the deposited polymer film is subjected to a plasma treatment which breaks down the polymer completely or partially.

6. Method according to one of Claims 1-5, **characterised in that** the substrate surface with the nanoparticles formed is subsequently introduced into a liquid phase which contains a solution of an inorganic metal compound, wherein dissolved material grows on the nanoparticles and thus enlarges the same and the substrate is pulled out of the liquid phase following a predetermined period of time, as a result of which a desired size of the nanoparticles is set.

7. Method according to one of Claims 1-6, **characterised in that** the intensity of the light field is set gradually or in steps in an extensive area by interference and diffraction on static or dynamically variable optical elements.

## Revendications

1. Procédé de production de nanoparticules ayant une taille latérale variable sur la surface d'un substrat, dans lequel un film polymérique, qui comprend au moins un composé métallique minéral, est déposé sur une surface du substrat et est ensuite irradié avec un champ lumineux latéral dans le plan à intensité modulée, moyennant quoi le composé métallique est réduit et est transformé en nanoparticules et la taille des nanoparticules situées à un endroit déterminé sur la surface du substrat est dépendante de l'intensité du champ lumineux à cet endroit, le film polymérique étant formé sur la surface du substrat par dépôt d'une solution micellaire d'un copolymère organique diblocs ou multiblocs, dont les micelles sont chargées avec au moins un composé métallique organique.

2. Procédé selon la revendication 1, **caractérisé en ce qu'**un gradient de la taille des particules est produit sur la surface du substrat.

3. Procédé selon la revendication 1 ou 2, **caractérisé en ce que** le film polymérique est déposé sur la surface du substrat de sorte qu'une structure ordonnée de domaines polymériques contenant des composés métalliques ayant une distance de séparation latérale déterminée soit produite, cette distance de séparation déterminant la distance de séparation des nanoparticules après la transformation des composés métalliques en nanoparticules.

4. Procédé selon la revendication 3, **caractérisé en ce qu'**un gradient de distance de séparation des domaines polymériques est produit sur la surface du substrat.

5. Procédé selon l'une des revendications 1 à 4, **caractérisé en ce que** le substrat doté du film polymérique déposé est soumis à un traitement plasma, qui dégrade entièrement ou partiellement le polymère, après irradiation avec la lumière.

6. Procédé selon l'une des revendications 1 à 5, **caractérisé en ce que** la surface du substrat dotée des nanoparticules formées est ensuite introduite dans une phase liquide, qui comprend une solution d'un composé métallique minéral, la matière dissoute croissant sur les nanoparticules et agrandissant donc celles-ci, et le substrat étant extrait de la phase liquide après un laps de temps prédéterminé, moyennant quoi une taille voulue des nanoparticules est ajustée.

7. Procédé selon l'une des revendications 1 à 6, **caractérisé en ce que** l'intensité du champ lumineux est ajustée dans le plan graduellement ou bien par paliers par interférence et diffraction sur des éléments optiques statiques ou modifiables sur le plan dynamique.
